**Europäisches Patentamt**

**European Patent Office**

**Office européen des brevets**

(19)

(11) Publication number: **0 099 183**
**A1**

(12)

# EUROPEAN PATENT APPLICATION

(21) Application number: **83303451.5**

(22) Date of filing: **15.06.83**

(51) Int. Cl.³: **C 07 H 15/04**

(30) Priority: **08.07.82 US 396228**

(43) Date of publication of application:
**25.01.84 Bulletin 84/4**

(84) Designated Contracting States:
**BE CH DE FR GB IT LI NL SE**

(71) Applicant: **A.E. STALEY MANUFACTURING COMPANY**
**2200 Eldorado Street**
**Decatur, Illinois 62525(US)**

(72) Inventor: **Short, Rolland William Phillip**
**2657 Gregory Court**
**Decatur Illinois(US)**

(74) Representative: **Marchant, James Ian et al,**
**Elkington and Fife High Holborn House 52/54 High**
**Holborn**
**London WC1V 6SH(GB)**

(54) Method for preparing organo glycosides.

(57) Organo glycoside ethers are prepared by reacting saccharide containing compositions with alcohols of 3 or more carbon atoms in the presence of 2 or more moles of water for each aldose unit mole. The method is particularly effective for converting polysaccharides, such as starches to alkyl glycosides by means of a reaction media comprised of starch, an alkanol of at least 3 carbon atoms and at least one other alcohol-soluble organo co-solvent in the presence of an acid catalyst at elevated temperatures under pressure.

EP 0 099 183 A1

## Method for Preparing Organo Glycosides

This invention relates to a method for preparing organo glycosides.

Numerous methods for preparing alkyl glycoside mixtures have been reported by the prior art. United States Patent No. 3,219,656 by Boettner discloses a typical process for preparing long-chain alkyl glycoside mixtures via a series of sequential alcohol interchange reactions. Boettner initially converts glucose and methanol to methyl glycoside, the methyl glycosides to butyl glycosides by a butanol interchange reaction and then the butyl glycoside mixture to a fatty glycoside mixture by a fatty alcohol interchange. Boettner suggests that water and the interchanged alcohol by-products should be removed during or prior to each successive interchange reaction. United States Patent No. 3,598,865 by Lew discloses a process for converting methyl glycoside to long-chain glucoside in the presence of primary and secondary alcohols of 3 to 5 carbon atoms. In another patent by Lew (U.S. Patent No. 3,974,138), butyl glycosides of a D.P. 1.1 to 4.0 are reportedly directly prepared by reacting glucose with n-butyl alcohol.

United States Patent No. 4,223,129 by Roth et al discloses a continuous process for making alkyl glycosides. The patentees studied the effects that feed starch moisture (2-11%) had upon methyl glycoside production. Dextrose levels were shown to increase as the residual moisture content increases. Roth et al concluded that methyl glycosides could be prepared without predrying the starch.

According to the present invention, there is provided a method of converting saccharides and alcohol into organo glycoside mixtures, characterised in that a saccharide is reacted with an organo alcohol of at least 3 carbon atoms under superatmospheric pressure and at a temperature sufficient to convert the saccharide and alcohol into an organo glycoside mixture, the reaction being carried out in the presence of at least 2 moles water for each saccharide molar unit and an effective amount of catalyst, and at least a portion of the organo glycoside mixture is recovered from the reaction mixture.

Generally the saccharide is dispersed into a liquid dispersant system comprising the organo alcohol as the major component thereof. The liquid dispersant system may also contain an alcohol-soluble organic co-solvent. The saccharide reacts with the alcohol which is the major component of the dispersant system to produce the organo glycoside mixture in which the organo group of the glycoside contains at least 3 carbon atoms.

The process generally applies to the preparation of organo glycoside mixture represented by the structural formula $R-O-(G)_x$ wherein "R", "G" and "x" respectively represent an organo group of at least 3 carbon atoms, a saccahride unit and the average number of saccharide units. The glycoside mixtures are prepared by reacting organic alcohols of at least 3 carbon atoms (e.g. alcohols of 3 to 25 carbon atoms) with the saccharide reactants under acid catalysis to produce the organo glycoside reaction product. The monohydric alcohols are well-suited for this purpose. Illustrative monohydric alcohols include the primary or secondary straight or branched saturated or unsaturated, alkyl, arylalkyl alcohols, ether alcohols, cyclic alcohols and heterocyclic alcohols. Advantageously the process is applied to reacting alkanols of 3 to 6 carbon atoms inclusive (e.g. propanol, butanol, pentanol, hexanol, mixtures thereof) with polysaccharides. The primary alkanols of 3 to 4 carbon atoms (preferably butanol) are particularly well-suited as the major (weight basis) liquid dispersant. The weight ratio of the major alcohol dispersant to the remaining liquid dispersants of the system will generally range from 2:1 to 5:1 and preferably from 3:1 to 4:1.

The value of "x" in the above formula can be controlled to a certain extent by the processing and reactant conditions. The more elevated temperatures and molar excesses of saccharide result in more extensive polymerization of glucosides and a higher "x" value. Conversely lower temperatures and molar excesses of the alcohol reactant reduces the extent of glycosidation. Although "x" may have a value ranging from 1 to 50 or higher, "x" will typically be of a value less than 20. Advantageously "x" will have a value within the 1 to 10 range and preferably less than 5.

The process applies generally to any saccahride-containing composition which can be converted into organo glycosides under acidic conditions with the liquid dispersant system of this invention. Such saccahride-containing compositions include compositions which molecularly contain at least one saccharide moiety or unit. Unsubstituted or substituted monosaccharides (e.g. aldose or ketoses) or polysaccharide (e.g. aldoside or ketosides) containing sugar alcohol or sugar ether units which are reactive with the three carbon and higher alcohols to form alkyl glycoside mixtures may be used for this purpose. Illustrative monosaccharides include aldoses such as apiose, arabinose, galactose, glucose, lyxose, mannose, gallose, altrose, isode, arabinose, ribose, talose, xylose, the lower aldose ethers thereof (e.g. methoxy-, ethoxy-, propoxy-, butoxy-), polyoxyalkylene (e.g. polyoxyethylene or polyoxypropylene) aldose ether derivatives which contain terminal hydroxy or lower alkoxy $(C_1-C_4)$ groups and mixtures thereof. Illustrative polysaccharides include disaccharides (e.g. maltose, lactose and sucrose), trisaccharides (e.g. maltotriose) saccharides of 4 or more saccharide units (e.g. hemicellulose, inulin, dextrin, dextran, xylan, starch and hydrolyzed starch and low D.E. corn syrup) the polysaccharide ester and ether derivatives (such as mentioned above) and mixtures thereof. The process of the present invention is especially adpated to polysaccharides and particularly to starches.

In addition to the major alcohol dispersant, it is advantageous to include in the reaction media at least one other alcohol-soluble organic solvent. A variety of non-polar (e.g. petroleum ether), or polar alcohol-soluble organic solvents may be used for this purpose. Polar solvents which are normally liquid at 20°C and form aqueous solutions when combined with an equal weight of water are particularly useful as the alcohol-soluble organic solvent. Illustrative alcohol-soluble organic solvents include water-miscible alcohols of 1 or 2 carbon atoms, ketones, aldehydes and mixtures thereof. Homogeneous alcoholic aqueous solutions comprised of water-miscible and alcohol-soluble organo solvents such as acetone, ethylene glycol, methanol, ethanol in combination with water and the major dispersant are advantageously used as the liquid dispersant system of this invention.

In addition to the major and minor organic solvents, the liquid dispersant system contains water. Commercially available starches will typically contain from 1.1 to 1.4 moles water per anhydrous glycoside mole or 10% to 13% water. To effectively use such starches herein, additional water needs to be added to the system. The total water content within the reaction media will typically be maintained at a level of at least 2.0 moles of water for each saccharide unit mole. The water content of the system includes the water contributed by the saccharide, other reactants and solvent dispersants as well as any added water. The water content in the reaction media can range from 2 to 25 moles for each saccharide unit mole. Advantageously the water content within the reaction media will range from 3 to 10 moles and preferably from 4 to 8 moles for each saccharide unit mole.

The reaction is conducted in the presence of a catalyst. Representative catalysts include inorganic or organic acids, acid electron accepting compounds such as the Lewis acids (e.g. boron, trifluoride, tin tetrachloride, aluminium chloride) and mixtures thereof. Illustrative acid catalysts include the strong mineral acids such as hydrochloric, hydrofluoric, hydriodic, phosphoric, sulfuric, sulfonic acids and mixtures thereof. Exemplary strong organic acid catalysts include the phosphonic or sulfonic acid derivatives of alkyl, alkylaryl, aryl, cyclic and heterocyclic organic compounds and mixtures thereof. Representative strong organic acids are the alkyl phosphonic acids and alkyl sulfonic acids such as the methyl, ethyl, butyl, propyl, amyl, sulfonic or phosphonic acids, phenyl sulfonic acid, phenyl phosphonic acid, paratoluene sulfonic acid, paratoluene phosphonic acid and mixtures thereof.

Resin catlaysts may also be used as converting catalysts. Similar to the organic catalysts, these resins will normally contain sulfonic and/or phosphonic acid groups. Certain of the sulphonic phenol-formaldehyde and polystyrene exchange cationic resins, which tend to thermally decompose at temperatures of 120°C or higher, are generally ineffective at processing temperatures of 130°C or higher. Thermally stable, acid resins such as a perfluorinated copolymeric resin (e.g. tetrafluoroethylene/perfluoro-3,6-dioxa-4-methyl-7-octenesulfonic acid commercially available from E.I. duPont as NAFION-H) and the like (e.g. see Chemical & Engineering News, March 15, 1982, pages 22-25) can be effectively adpated to those processes conducted at 140°C or higher.

The conversion of the saccharide and organo alcohol into the $R-O-(G)_x$ glycoside is typically conducted at elevated temperatures and pressures. The reaction will most generally be conducted at a temperature of 100°C or higher and most typically within the 120°C to 200°C range.

-6-

The preferred operational temperature for reacting starch with the $C_3$-$C_4$ alkanols ranges from 140°C to 180°C.

The reaction may be conducted in batch or continuous reactors designed to maintain the dispersants in a liquid state under superatmospheric pressure and at the elevated reaction temperatures. The reactors are suitably equipped or designed to provide sufficient agitation to uniformly admix the reactants and facilitate pasting of the polysaccahride. In continuous reactor systems (e.g. continuous tubular reactors, helical coils, pipes, etc.), utilization of positive displacement pumps will generally provide sufficient agitation for this purpose. Such displacement pumps also afford a means for metering into the feed stream the appropriate reactant and liquid dispersant levels and regulating residence time and flow rates through the reaction zone. In such systems, the reaction media is continuously forced through the reaction zone under positive pressure by continuously metering into the feed stream the required reactants, liquid dispersant and catalyst concentration for the conversion reaction. Propeller, turbine, sweep, anchor or other appropriate agitating means, are generally applied to batch reactor systems. The reactors (continuous or batch) are also suitably equipped with heating and cooling means (e.g. steam, mineral baths) to maintain the reactants and reaction products at the appropriate processing temperature. If desired, continuous reactors may be constructed so as to provide a plurality of zones operated at different temperatures. Zones operated at reduced temperatures, following the polysaccharide pasting step, may be designed into the reactor system. The catalyst may be incorporated into the feed slurry or alternatively as a fixed catalyst bed.

After the conversion reaction has been completed, the chemical reaction may be terminated by conventional techniques such as cooling, by adding base to neutralize the catalyst, removal of the reaction product from the catalyst system, etc. If desired, the glycoside mixture may be fractionated such as by adding a precipitating solvent, concentrating and/or cooling to selectively cyrstallize one or more of the glycoside components from the reaction product, etc.

Volatile components may be suitably stripped from the glycoside mixture or fractionated by conventional techniques (e.g. flash-cooling, distillation, vacuum distillation), recycled and reused as liquid dispersant components. If a particular alkyl glycoside fraction is sought, the desired fraction may be recovered and the balance recycled to the reactor feed stream. Since the reaction is generally an equilibrated chemical reaction, such recycling can be effectively used to conserve upon the total reactant and liquid dispersant requirements for the alkyl glycoside manufacture by driving the reaction towards the desired end-product production. The recovered lower alkyl glycosides may be used as an intermediate glycoside component in the manufacture of glycosidic surfactants.

The liquid dispersant system permits the alcoholysis of polysaccharides to be conducted at lower processing temperature within a shorter reaction time interval and at a substantially lower viscosity. Effective alkyl glycoside production requires the polysaccharide to be sufficiently dispersed so that it may then be interchanged with the alcohol reactant. The present invention provides a means for effectively pasting starches and conducting the alcoholysis reaction at a reduced temperature. The lower reaction temperature reduces the level of undesirable reaction by-products and repolymerization of the lower D.P. glycosides into the higher D.P. oligosaccharide reaction products.

Another advantage of the present invention is that the process can be conducted at lower viscosities than normal for polysaccharide reactants.. These reduced working viscosities permit a broader range of processing equipment to be effectively used in the alkyl glycoside manufacture. Product yields and quality are also facilitated by avoiding excessively viscous reaction conditions.

Many polysaccharides, such as starch, when subjected to elevated temperatures and shear will exhibit an increase in viscosity until the starch pasting temperature has been achieved, after which the solution viscosity will decrease as the pasted polysaccharide components become uniformly dispersed or dissolved throughout the reaction medium. The pasting temperature, time interval required to achieve the peak viscosity and the peak viscosity duration are directly related to the efficacy of the liquid dispersant system. The liquid dispersant system of this invention effectively reduces both the peak viscosity and its duration. In a continuous operation conducted under pressurized or superatmospheric conditions, the starch reactants attain a peak viscosity (at an acceptable viscosity level) within a short time interval and readily disperse within the media at a sufficiently low solution viscosity level to permit effective interchange with the higher alcohol reactant.

In a conventional process, a low concentration of polysaccharide is usually required to provide a reaction media of an acceptable and manageable viscosity level. The liquid dispersant system of this invention permits the manufacturer to significantly increase the polysaccharide concentration while still operating at a manageable solution viscosity level. This permits the manufacturer to achieve greater yields and productivity. Reactor fouling and the need for specialized process equipment to handle excessively viscous materials at elevated temperatures are alleviated through the novel liquid dispersant systems of this invention.

The following Examples illustrate the use of this invention to prepare alkyl glycosides:

### EXAMPLE I

A butyl glucoside mixture was prepared (Run A) by slurrying 15.00 parts by weight (dry substance basis) corn starch (PFP) in 84.74 parts by weight of a liquid dispersant consisting of 79.4% anhydrous butanol, 11.8% water and 8.8% anhydrous methanol (on a weight basis) and 0.26 parts by weight p-toluenesulfonic acid monohydrate catalyst (15 meq per mole of starch). The total water content, including that provided by the starch (12.0% by weight water) and added water, was 9.96% of the total reaction mixture. A 2-liter stainless steel Parr reactor, equipped with an internal stainless steel heating and cooling coil, a thermocouple immersed in a glycerine well and operatively connected to a pyrometer to record the reaction temperature, a pressure release valve and two (three-bladed propeller) stirrers mounted on a single shaft driven by a constant speed electric motor with matching Servodyne constant speed and torque control unit No. 4425 and recorder to record power changes in millivolts (mV) was used as the reactor.

The reaction slurry was rapidly heated (steam at 140 psig) and continually stirred at 900 rpm. Upon reaching 155°C (about 4 minutes after heating), the viscosity suddenly increased to the maximum recorded peak viscosity level of 172 mV. The 155°C temperature represents the peak viscosity temperature or the starch pasting temperature. Within two additional minutes (6 minute total time), the viscosity rapidly decreased to a level comparable to that of the original reaction slurry. The reaction was held at 160-165°C (steam pressure of 115 psig) for 10 minutes and then cooled within 14 minutes to 30°C.

The resultant light yellow reaction product (1066 g, 98.7% recovery) was filtered to remove trace amounts of insolubles. The catalyst was then neutralized (pH 7.0) by mixing a 124.63g aliquot of the filtrate (pH 2.55) with 50 ml deionized water and titrating with 16.80 ml of 0.1N sodium hydroxide. The sample was then adjusted to pH 8.55 and allowed to quiescently stand for at least 16 hours and the pH was then readjusted from pH 6.9 to pH 8.5 with 0.10N sodium hydroxide. The free solvents (butanol, methanol and water) were then vacuum distilled (83-85°C at 19.25.5 inches mercury vaccum) from the sample. A small amount of residual butanol (2-3%) was then removed by redissolving the butyl glucoside residue in deionized water (100 ml) and vacuum distillation. The total solids in solution and yields were calculated. Butyl glucoside distributions were determined on an aqueous solution of the residue by using HPLC (high performance liquid chromatography). Glucose content was determined on the original filter sample via the auto-analyzer method.

For comparative purposes, Run A was repeated except that a 12% moisture starch (Run B) and a 5.8% moisture starch (Run C) were used without any additional water being added to the reaction media. Run (A) was also repeated by Runs (D), (E) and (F) using different levels of butanol/methanol/water as recorded in Table 1. The reaction media for all the Runs were comprised of 15.00% starch dry substance, 84.74% solvent (water plus n-butanol and methanol in a 90:10 ratio) and 0.26% p-toluenesulfonic acid monohydrate. Table 1 summarizes the results of this study.

## TABLE 1

### Effect of Water Level in the Reaction

| | RUN A | RUN B | RUN C | RUN D | RUN E | RUN F |
|---|---|---|---|---|---|---|
| Butanol, % | 67.29 | 74.43 | 75.44 | 71.49 | 63.54 | 60.20 |
| Methanol, % | 7.47 | 8.27 | 8.38 | 7.95 | 7.07 | 6.69 |
| Water, % | 9.96 | 2.04 | 0.92 | 5.29 | 14.13 | 17.85 |
| Water, moles per mole starch | 5.98 | 1.22 | 0.55 | 3.17 | 8.48 | 10.71 |
| Butanol/methanol/water weight ratio | 79/4/8.8/11.8 | 87.8/9.8/2.4 | 89.0/9.9/1.1 | 84.4/9.4/6.2 | 75.0/8.3/16.7 | 71.0/7.9/21.1 |
| Reaction Temp °C for 10 min | 160-165 | 171-176 | 176-179 | 168-172 | 156-162 | 155-160 |
| **Peak viscosity** | | | | | | |
| mV | 172 | 500 | 600 | 265 | 130 | 114 |
| °C | 155 | 170 | 176 | 164 | 149 | 144 |
| Duration, sec. | 120 | 130 | 180 | 120 | 90 | 84 |
| **Solution Color** | | | | | | |
| Visual | Lt. yellow | Red | Red | Yellow | Lt. yellow | Lt. yellow |
| Light Absorbance @ 450 nm | 0.12 | 0.38 | 0.79 | 0.23 | 0.09 | 0.07 |
| Total solids | 20.9% | 21.0% | 20.4% | 21.4% | 20.6% | 20.6% |
| Yield based on starch | 138% | 138% | 134% | 141% | 135% | 135% |
| **Butyl Glucoside Distribution, %** | | | | | | |
| Mono- | 53 | 59 | 59 | 56 | 48 | 43 |
| Di-[a] | 22 | 23 | 24 | 23 | 21 | 21 |
| Tri-[b] | 5 | 4 | 4 | 5 | 7 | 8 |
| Higher[c] | 8 | 9 | 9 | 8 | 8 | 8 |
| Glucose, % | 12 | 5 | 4 | 8 | 17 | 21 |

a – 10% of which is methyl monoglucoside;   b – 10% of which is methyl diglucoside;   c – 10% of which is methyl tri and higher glucosides

As illustrated by the Table 1 results, excesses in water significantly reduces the peak viscosity, the peak viscosity temperature and the peak viscosity duration. The butyl glycosides prepared with more than 3 moles water for each saccharide unit mole collectively exhibited lighter colors than those made with less than 3 moles water. At the 17.85% water level (Run F), a slight separation of the aqueous phase occured. A homogeneous solution was obtained by increasing the starch dry substance level to 17.5 %. A slight separation of the aqueous phase was also obtained using a starch dry substance level of 16.8% and a butanol/methanol/water ratio of 67.5/7.5/25.0.

## EXAMPLE II

In this Example 15.14% PFP corn starch dry substance 84.60 % solvent (butanol plus water), and 0.26% p-toluenesulfonic acid monohydrate were used to prepared butyl glycosides. Except as indicated in Table 2, the butyl glycosides were prepared pursuant to the procedure of Example I. Table 2 records the results.

TABLE 2

## Effect of Water Level Using Butanol as the Solvent

| | RUN G | RUN H | RUN I | RUN J | RUN K | RUN L |
|---|---|---|---|---|---|---|
| Butanol, % | 74.66 | 70.89 | 66.96 | 63.07 | 59.17 | 42.32 |
| Water, % | 9.95 | 13.75 | 17.64 | 21.53 | 25.42 | 42.28 |
| Butanol/water ratio | 88.2/11.8 | 83.8/16.2 | 79.1/20.9 | 74.6/25.4 | 69.9/30.1 | 50/50 |
| Water, moles per mole starch | 5.91 | 8.17 | 10.48 | 12.79 | 15.10 | 25.12 |
| Reaction Temp. for 13 min °C | 166-169 | 166-170 | 166-169 | 157-170 | 163-168 | 166-167 |
| **Peak viscosity** | | | | | | |
| mV | $220^d$ | 140 | 80 | 34 | 26 | 25 |
| Temp. °C | 156 | 155 | 149 | 148 | 140 | 100 |
| Duration, sec. | 120 | 84 | 54 | 48 | 36 | 108? |
| Butyl Glucoside Distribution in Total Product, % | | | | | | |
| Mono- | 63 | 59 | 56 | 52 | 47 | 32 |
| Di- | 11 | 10 | 10 | 10 | 7 | 4 |
| Tri- | 5 | 5 | 6 | 6 | 7 | 9 |
| Others | 8 | 9 | 8 | 7 | 8 | 8 |
| Glucose, % | 13 | 17 | 20 | 25 | 31 | 47 |

d - 172 mV when 10% of the butanol was replaced for a butanol/methanol/water ratio of 79.4/8.8/11.8 (Run A)

Under the processing conditions of this Example, phase separation occurred when the total water content was increased to 17.64% or higher (e.g. see Runs I-L). The yield of product in the water phase proportionally increased as the water levels increased until the 42.28% level of Run L. The carbohydrate distributions found in the butanol and water phases for the Run I-L products were similar as shown below.

| RUNS I-L | BUTANOL PHASE | WATER PHASE |
|---|---|---|
| Butyl Monoglucoside, % | 59-66 | 7-14 |
| Butyl Diglucoside, % | 7-11 | 3-11 |
| Butyl Triglucoside, % | 2-5 | 12-22 |
| Others, % | 7-11 | 4-10 |
| Glucose, % | 12-19 | 55-64 |

### EXAMPLE III

Butyl glycosides were prepared at varying starch concentrations by employing the reaction conditions of Run A (except as indicated in Table 3) with the starch being dispersed into butanol/methanol/water of a weight ratio of 79.4/8.8/11.8, except for the 77.4/8.4/14.2 ratio of Run Q. Runs A, M and N were conducted at 15 meq catalyst per starch mole level with Runs O, P and Q at the 10 meq level. The results are tabulated in Table 3.

TABLE 3

Effect of Starch Concentration Upon the Reaction

| | RUN A | RUN M | RUN N | RUN O | RUN P | RUN Q |
|---|---|---|---|---|---|---|
| Starch solids, % | 15 | 17 | 19 | 21 | 24 | 28 |
| Liquid Dispersant, % | 84.74 | 82.70 | 80.67 | 78.75 | 75.71 | 71.67 |
| P-toluenesulfonic acid.$H_2O$, % | 0.26 | 0.30 | 0.33 | 0.25 | 0.29 | 0.33 |
| Water, moles per AGU | 5.98 | 5.15 | 4.49 | 3.92 | 3.27 | 3.27 |
| **Peak viscosity** | | | | | | |
| mV | 172 | 165 | 200 | — | — | — |
| Temp. °C | 155 | 159 | 157 | 159 | 157 | 155 |
| Duration, sec | 120 | 60 | 120 | — | — | — |
| Reaction Temp., °C | 160-165 | 167-170 | 166-170 | 159-162 | 157-163 | 155-163 |
| Reaction, Time, min. | 10 | 13 | 13 | 22 | 20 | 25 |
| Reaction Solution, Color | Light Yellow | Sl dark Yellow | Dark Yellow | Light Yellow | Yellow | Reddish Yellow |
| Total Solids | 20.9% | 23.3% | 25.2% | 28.5% | 32.3% | 35.9% |
| Yield based on starch | 138% | 135% | 132% | 133% | 130% | 124% |
| Brookfield RVT viscosity (cps) | 13 | 15 | 16 | 19 | 22 | 25 |
| Butyl Glucoside Distribution, % | | | | | | |
| Mono- | 53 | 49 | 51 | 47 | 46 | 44 |
| Di-[a] | 22 | 22 | 22 | 22 | 24 | 23 |
| Tri-[b] | 6 | 7 | 7 | 9 | 10 | 11 |
| Others[c] | 7 | 9 | 7 | 10 | 8 | 9 |
| Glucose, % | 12 | 13 | 13 | 12 | 12 | 13 |

a - Supra;   b - Supra;   c - Supra.

0099183

-16-

As illustrated by the Table 3 data, increasing the starch solids level from 15 to 28% (1) increased the peak viscosity without changing the peak viscosity temperature, (2) gave more highly colored reaction solutions, (3) decreased yields from 138% to 124%, and (4) increased the Brookfield viscosity from 13 to 25 cps. The overall composition (glucose and butyl glucosides), however, remained unaffected by starch solids level.

### EXAMPLE IV

This Example illustrates that other alcohol-soluble co-solvents may be effectively used as a co-solvent or replacement for the methanol of Run A. In this Example, the reaction media contained 15.1% corn starch dry substance, 67.2% butanol, 9.9% total water, 7.5% alcohol-soluble co-solvent (as identified in Table 3) and 0.3% p-toluenesulfonic acid. Except as otherwise indicated by this Example, the butyl glycoside preparation conformed to that of Run A.

The results of this Example are recorded in Table 4.

## TABLE 4

| | RUN G | RUN A | RUN R | RUN S | RUN T | RUN U |
|---|---|---|---|---|---|---|
| Alcohol-Soluble co-solvent | None | Methanol | Ethanol | Ethylene Glycol | Acetone | Skelly-solve F |
| **Peak viscosity** | | | | | | |
| mV | 220 | 172 | 234 | 133 | 180 | 118 |
| Temp. °C | 156 | 155 | 160 | 153 | 155 | 151 |
| Duration, sec. | 120 | 120 | 108 | 60 | 144 | 108 |
| Reaction Temp. 10-13 min, °C | 166-169 | 160-165 | 167-169 | 165-169 | 156-169 | 156-170 |
| Color | yellow | light yellow | yellow | yellow | Sl.dark yellow | red |
| Total Solids | 22.8% | 20.9% | 21.7% | 20.1% | 21.6% | 21.8% |
| Yield based on starch | 149% | 138% | 141% | 132% | 141% | 137% |
| Butyl Glucoside Distribution % | | | | | | |
| Mono- | 63 | 53 | 55 | 58 | 61 | 62 |
| Di- | 11 | 22 | 16 | 17 | 11 | 13 |
| Tri- | 5 | 6 | 5 | 6 | 5 | 5 |
| Others | 8 | 7 | 10 | 5 | 9 | 5 |
| Glucose, % | 13 | 12 | 13 | 14 | 14 | 14 |

Higher peak viscosities were obtained when methanol was omitted (Run G) or replaced by either ethanol (Run R) or acetone (Run T). However, lower peak viscosities were obtained by substituting either ethylene glycol (Run S) or Skellysolve F (Run U) for methanol.

### EXAMPLE V

This Example illustrates the effect of stirring upon the reaction product. The liquid dispersant system for this Example (butanol/methanol/water at 79.4/8.8/11.8) was prepared by slurrying 15.1% PFP corn starch in 67.2% butanol, 7.5% methanol, 10.0% water and 0.18-0.33% p-toluene sulfonic acid.$H_2O$. The results are tabulated in Table 5.

## TABLE 5

|  | RUN V | RUN W | RUN X | RUN A | RUN Y |
|---|---|---|---|---|---|
| Stirrer speed, rpm | 500 | 700 | 800 | 900 | 1200 |
| **Peak viscosity** |  |  |  |  |  |
| mV | 142 | 116 | 124 | 172 | 168 |
| Temp. °C | 156 | 156 | 157 | 155 | 159 |
| Duration, sec | 48 | 24 | 48 | 120 | 30 |
| Reaction time at 160-169°C, min | 13 | 11 | 12 | 10 | 13 |
| Insoluble residue on starch | 41% | 29% | 0.15% | trace | 0.21% |
| Total solids | 16.7% | 18.5% | 20.8% | 20.9% | 22.3% |
| Yield based on starch | 102% | 115% | 135% | 138% | 143% |
| **Butyl Glycoside distribution, %** |  |  |  |  |  |
| Mono- | 50 | 50 | 52 | 53 | 50 |
| Di-[a] | 22 | 23 | 22 | 22 | 24 |
| Tri-[b] | 6 | 7 | 6 | 6 | 7 |
| Others[c] | 11 | 8 | 8 | 7 | 8 |
| Glucose, % | 11 | 12 | 12 | 12 | 11 |

a - Supra;  b - Supra;  c - Supra.

Under the processing conditions of this Example, stirring speeds of 500 rpm (Run V) and 700 rpm (Run W) yielded relatively large amounts (41% and 29%) of pasted but insoluble starch residues. The higher stirring speeds of 800 rpm (Run X) to 1200 rpm (Run Y) significantly reduced insoluble starch residues.

### EXAMPLE VI

This Example illustrates the versatility of the process to different catalyst systems using the liquid dispersant system of Run A. In this Example, 15.0%, PFP corn starch dry substance was slurried in about 67.5% butanol, 7.5% methanol, and 10% water containing from about 0.24-0.88% catalyst. Table 6 records the results of this Example.

TABLE 6

| Catalyst | RUN A | RUN Z | RUN AA | RUN AB | RUN AC |
|---|---|---|---|---|---|
| | P-TSA.$H_2O$ | $H_2SO_4$ | p-TSA.$H_2O$ | MSA | $H^+$Resin |
| meq. | 15 | 15 | 18.4 | 18.4 | 31.8 |
| **Peak Viscosity** | | | | | |
| mV | 172 | 118 | 156 | 102 | 200 |
| Temp, °C | 155 | 156 | 159 | 155 | 167 |
| Duration, sec | 120 | 108 | 90 | 60 | 228 |
| Reaction time at 169-170°C, min | 10 | 13 | 13 | 13 | 20 |
| Insoluble Residue % on starch | trace | trace | 0.06 | 0.08 | 90 |
| Reaction solution color | Lt.Yellow | Lt.Yellow | Yellow | Yellow | Yellow |
| Total Solids | 20.9% | 20.6% | 21.8% | 21.1% | 1.45% |
| Yield, based on starch | 183% | 135% | 143% | 138% | 9.7% |
| **Butyl Glucoside, Distribution, %** | | | | | |
| Mono-[a] | 53 | 52 | 52 | 52 | 25 |
| Di-[b] | 22 | 23 | 23 | 21 | 21 |
| Tri-[c] | 6 | 5 | 5 | 5 | 14 |
| Other | 7 | 8 | 8 | 9 | 25 |
| Glucose, % | 12 | 12 | 12 | 13 | 15 |

a - Supra;  b - Supra;  c - Supra.

In this Example the catalyst employed for Runs A and AA was p-toluene sulfonic acid monohydrate, sulfuric acid for Run Z, methane sulfonic acid for Run AB and Dowex 50WX12 in the acid form for Run AC. The catalyst milliequivalents are based upon 162 g anhydrous glucose unit (1.0 mole) of the starch.

## EXAMPLE VII

The versatility of the process to different carbohydrate reactants using the liquid dispersant system of butanol/methanol/water at a ratio of 79.4/8.8/11.8 is illustrated by this Example. In each of the Runs, 15.1% carbohydrate (dry substance basis) was slurried in 67.2% butanol, 7.5 % methanol, 10.0% water with 0.3% p-toluenesulfonic acid monohydrate. The methodology of Run A was followed except as noted in Table 7.

## TABLE 7

### Effect of Carbohydrate Moiety on the Reaction

| Carbohydrate | RUN A | RUN AD | RUN AE | RUN AF | RUN AG |
|---|---|---|---|---|---|
| | PFP Corn Starch | Waxy Maize Starch | Dextrin[e] (White) | Acid Thinned Starch | Cellulose[f] |
| **Peak Viscosity** | | | | | |
| mV | 172 | 173 | 104 | 94 | None |
| Temp, °C | 155 | 155 | 148 | 160 | None |
| Duration, sec | 120 | 48 | 48 | 60 | None |
| Reaction time at 160–174°C, min | 10 | 13 | 13 | 12 | 20 |
| Insoluble residue, % on carbohydrate | trace | trace | 0.1 | 29 | 94 |
| Reaction Solution color | Lt.Yellow | Yellow | Dk.Yellow | Yellow | Lt.Yellow |
| Total Solids | 20.9% | 21.2% | 20.7% | 18.0% | 0.8% |
| Yield based on carbohydrate | 138% | 138% | 135% | 112% | 5.6% |
| **Butyl Glucoside Distribution** | | | | | |
| Mono-[a] | 53 | 51 | 51 | 49 | 36 |
| Di-[b] | 22 | 22 | 22 | 23 | 14 |
| Tri-[c] | 6 | 5 | 5 | 7 | 22 |
| Other | 7 | 9 | 9 | 10 | 24 |
| Glucose, % | 12 | 13 | 13 | 11 | 4 |

a – Supra;  b– Supra;  c – Supra;  e – STADEX 60K, manufactured and distributed by the A.E. Staley Manufacturing Company, Decatur, Illinois, U.S.A.;  f – Solka Floc – manufactured and distributed by Dicalite Div. Grefco., Inc., Los Angeles, California, U.S.A.

The glycoside distributions of the reaction products for the waxy maize starch, white dextrin and acid-thinned starch hydrolyzate Runs were similar to those obtained from corn starch under Run A. The white dextrin (Run AE) had a lower peak viscosity but yielded a darker colored reaction solution. The acid thinned starch product (Run AF) also had a lower peak viscosity but not all the starch reacted with the alcohol as indicated by the 29% yield of insoluble residue. Similar results are also obtained by replacing the corn starch with an equivalent amount of dextrose or methyl glucoside.

EXAMPLE VIII

In this Example 173 grams of starch was slurried into 668.6 grams butanol, 74.3 grams methanol with a sufficient amount of water being added to provide a total of 98.7 grams water. Three (3) grams P-toluenesulphonic acid catalyst was used. Run AH was conducted in accordance with Run A except as indicated in Table 8. Run AI was conducted by adding an additional 173 grams of starch (d.s.b. - 1.07 M) to the filtrate of Run AH. In Run AJ an additional 173 grams of starch was then added to the Run AI filtrate. Table 8 records this study.

TABLE 8

|  | RUN AH | RUN AI | RUN AJ |
|---|---|---|---|
| Moles water/ starch mole | 5.7 | 5.7 | 5.7 |
| Peak viscosity |  |  |  |
| mV | 165 | 120 | 64 |
| Temp. °C | 159 | 151 | 151 |
| Duration, Sec. | 60 | 144 | 300 |
| Reaction Temp, °C | 164-165 | 156-160 | 153-159 |
| Reaction Time, Min. | 11 | 13 | 13 |
| Reaction solution color | Reddish-yellow | Reddish | Dark Red |
| Total Solids | 23.4% | 36.8% | 47.2% |
| Yield based on starch | 135% | 128% | 127% |
| Butyl Glucoside Distribution, % |  |  |  |
| Mono- | 53 | 44 | 37 |
| Di-[a] | 25 | 24 | 23 |
| Tri-[b] | 6 | 11 | 14 |
| Others[c] | 5 | 10 | 14 |
| Glucose, % | 11 | 11 | 12 |

a - Supra;  b - Supra;  c - Supra.

As illustrated by the Table 8 data, PFP corn starch can be reacted with butanol (Run AH) then the resultant excess butanol in the butyl glucoside reaction product can be further reacted with an additional molar equivalency of 173 gram starch (Run AI) followed by the addition of another 173 grams of starch to the reaction product of Run AI. A solution colour increase with a slight decrease in yields was observed with each successive run. The concentration of monoglucoside decreases from 53% to 37% while the triglucoside content increases from 6% to 15%. The change in $D.P._4$ and higher saccharide indicates that butyl glucoside made from the previous runs further polymerized into higher DP butyl polyglucosides.

## EXAMPLE IX

Other alkyl glucoside, besides n-butyl glucoside, can be prepared by reacting PFP corn starch directly with the alcohol. In this example, isopropyl glucoside was prepared by reacting 19.00 parts of starch dry substance with 69.43 parts (by weight) of isopropanol (9.85 moles per mole of starch) in the presence of 11.23 parts of total water (5.32 moles per mole of starch) and 0.335 parts of p-toluenesulfonic acid monohydrate. The reaction was carried out using the equipment and procedure of Example I with the following reaction parameters and results: peak viscosity of 254 mV, peak viscosity temperature of 156°C., peak viscosity duration of 300 seconds, and reaction temperature cf 164-169°C. for 13 minutes. A trace amount of insoluble material was removed from the reddish isopropyl glucoside solution. The solution contained 23.50% total solids. The total solids yield (on starch d.s.b.) was 124%. The product contained 19% glucose and the isopropyl glucoside distribution was 49% mono-, 18% di-, 3% tri- and 11% others.

CLAIMS:

1.    A method for converting saccharides and alcohol into organo glycoside mixtures, characterised in that a saccharide is reacted with an organo alcohol of at least 3 carbon atoms under superatmospheric pressure and at a temperature sufficient to convert the saccharide and alcohol into an organo glycoside mixture, the reaction being carried out in the presence of at least 2 moles water for each saccharide molar unit and an effective amount of catalyst, and at least a portion of the organo glycoside mixture is recovered from the reaction mixture.

2.    A method as claimed in claim 1 characterised in that the organo alcohol comprises the major liquid component of the reaction medium and the reaction medium additionally contains at least one other alcohol-soluble organic co-solvent.

3.    A method as claimed in claim 2 characterised in that the alcohol-soluble organic co-solvent is at least one of acetone, ethylene glycol, methanol and ethanol.

4.    A method as claimed in claim 2 or 3 characterised in that the major liquid component of the reaction medium comprises a monohydric alcohol.

5.    A method as claimed in claim 4 characterised in that the monohydric alcohol comprises an alkanol containing from 3 to 5 carbon atoms inclusive.

6.    A method as claimed in any of claims 1 to 5 characterised in that the liquid dispersant contains from 2 to 6 moles water for each anhydrous glucose mole and the reaction is conducted in a tubular reaction zone at a temperature ranging from 120°C to 200°C.

7.    A method as claimed in any of claims 1 to 6 characterised in that the polysaccharide consists essentially of a starch and the liquid dispersant consists essentially of butanol, water and methanol.

8.    A method as claimed in claim 7 characterised in that the weight ratio of butanol to total water and methanol of the liquid dispersant ranges from 3:1 to 4:1.

9.    A method as claimed in claim 8 characterised in that the reaction is conducted at a temperature ranging from 140 to 180°C.

10.   A method as claimed in claim 3 characterised in that the weight ratio of monohydric alcohol to the total water and co-solvent weight ranges from 2:1 to 4:1, the monohydric alcohol comprises an alkanol of 3 to 4 carbon atoms, the saccharide comprises starch and the conversion reaction is conducted at a temperature ranging from 140°C to 180°C.

European Patent
Office

**EUROPEAN SEARCH REPORT**

Application number

EP 83 30 3451

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int. Cl. ³) |
| Y | US-A-2 390 507 (S.M. CANTOR)<br>* Page 3, example 3; pages 4-6 *<br>--- | 1-14 | C 07 H 15/04 |
| Y,D | US-A-4 223 129 (C.D. ROTH)<br>* Claims 1-8 *<br>----- | 1-14 | |
| | | | **TECHNICAL FIELDS SEARCHED (Int. Cl. ³)**<br><br>C 07 H 15/00 |

The present search report has been drawn up for all claims

| Place of search<br>THE HAGUE | Date of completion of the search<br>14-09-1983 | Examiner<br>VERHULST W. |
|---|---|---|